(19)

**Europäisches Patentamt**
**European Patent Office**
**Office européen des brevets**

(11) **EP 3 074 775 B1**

(12) **EUROPEAN PATENT SPECIFICATION**

(45) Date of publication and mention of the grant of the patent:
**11.07.2018 Bulletin 2018/28**

(21) Application number: **14805577.5**

(22) Date of filing: **27.11.2014**

(51) Int Cl.:
*G01N 33/92* *(2006.01)*    *G01N 24/08* *(2006.01)*
*G01R 33/465* *(2006.01)*

(86) International application number:
**PCT/EP2014/075873**

(87) International publication number:
**WO 2015/079000 (04.06.2015 Gazette 2015/22)**

(54) **METHOD FOR THE CHARACTERIZATION OF LIPOPROTEINS**

VERFAHREN ZUR CHARAKTERISIERUNG VON LIPOPROTEINEN

PROCÉDÉS DE CARACTÉRISATION DE LIPOPROTÉINES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.11.2013 EP 13382478**

(43) Date of publication of application:
**05.10.2016 Bulletin 2016/40**

(73) Proprietors:
• **Institut d'Investigació Sanitària Pere Virgili**
  **43003 Tarragona (ES)**
• **Universitat Rovira I Virgili**
  **43003 Tarragona (ES)**

(72) Inventors:
• **MALLOL PARERA, Roger**
  **E-43204 Reus - Tarragona (ES)**
• **AMIGÓ GRAU, Núria**
  **E-43203 Reus - Tarragona (ES)**
• **CORREIG BLANCHAR, Xavier**
  **E-43203 Reus - Tarragona (ES)**
• **MASANA MARÍN, Lluís**
  **E-43005 Tarragona (ES)**
• **RODRÍGUEZ MARTÍNEZ, Miguel Ángel**
  **E-43480 Vilaseca, Tarragona (ES)**
• **HERAS IBÁÑEZ, Mercedes**
  **E-43202 Reus - Tarragona (ES)**
• **PLANA GIL, Núria**
  **E-43008 Tarragona (ES)**
• **RIBALTA VIVES, Josep**
  **Reus, Tarragona E-43202 (ES)**

(74) Representative: **ABG Patentes, S.L.**
**Avenida de Burgos, 16D**
**Edificio Euromor**
**28036 Madrid (ES)**

(56) References cited:
**WO-A1-93/03450      WO-A1-2005/043171**
**WO-A1-2005/119285**

• **ROGER MALLOL ET AL: "Human serum/plasma lipoprotein analysis by NMR: Application to the study of diabetic dyslipidemia", PROGRESS IN NUCLEAR MAGNETIC RESONANCE SPECTROSCOPY, vol. 70, 11 September 2012 (2012-09-11), pages 1-24, XP055108331, ISSN: 0079-6565, DOI: 10.1016/j.pnmrs.2012.09.001**
• **ROGER MALLOL ET AL: "Particle size measurement of lipoprotein fractions using diffusion-ordered NMR spectroscopy", ANALYTICAL AND BIOANALYTICAL CHEMISTRY, SPRINGER, BERLIN, DE, vol. 402, no. 7, 1 February 2012 (2012-02-01), pages 2407-2415, XP035016024, ISSN: 1618-2650, DOI: 10.1007/S00216-011-5705-9**
• **MARCUS STÅHLMAN ET AL: "Dyslipidemia, but not hyperglycemia and insulin resistance, is associated with marked alterations in the HDL lipidome in type 2 diabetic subjects in the DIWA cohort: Impact on small HDL particles", BIOCHIMICA ET BIOPHYSICA ACTA (BBA) - MOLECULAR AND CELL BIOLOGY OF LIPIDS, vol. 1831, no. 11, 27 July 2013 (2013-07-27), pages 1609-1617, XP055108648, ISSN: 1388-1981, DOI: 10.1016/j.bbalip.2013.07.009**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

## OBJECT OF THE INVENTION

The present invention relates to a method for the characterization of lipoproteins and is applicable in the field of biomedicine.

## PRIOR ART

Lipids are mainly present in the blood in the form of lipoproteins, which are particles synthesized in the liver and intestines that transport cholesterol, triglycerides and other lipids through the blood stream into peripheral tissues. Abnormal levels of blood lipids may be indicative of cardiovascular diseases. To assess cardiovascular risk, a standard lipid panel is generally used, which includes the concentration of plasma triglycerides, total cholesterol, LDL cholesterol and HDL cholesterol. All these parameters are measured experimentally except for the LDL cholesterol, which is estimated using the Friedewald formula. A critical limitation of this formula is its inaccuracy under certain conditions. Also, the measure of the amount of cholesterol in HDL and LDL lipoprotein fractions does not suffice to predict cardiovascular risk in every case, since also the size and particle number of lipoprotein particles may be relevant for a correct diagnosis of lipid-related diseases. Thus, lipoprotein particles play a main role in cardiovascular diseases and metabolic disorders.

Lipoproteins are divided into five main fractions depending on their size and density: chylomicrons (Q; radii 400-2500 Å), very low density lipoproteins (VLDL; radii 150-400 Å), intermediate density lipoproteins (IDL; radii 125-175 Å), low density lipoproteins (LDL; radii 90-140 Å) and high density lipoproteins (HDL; radii 25-60 Å). These main fractions can be further divided into different subclasses to obtain a more detailed lipoprotein profile.

It is generally accepted that the structure of a lipoprotein particle is substantially spherical and comprises an inner core and an outer shell, wherein non-polar lipids (triacylglycerols and cholesteryl esters) are found within the core, while polar lipids (phospholipids and free cholesterol) are distributed through a surface monolayer (shell). The protein components of lipoproteins (called apolipoproteins or apoproteins) are located on the shell together with the polar lipids.

Advanced lipoprotein testing (ALT) aims to provide the most detailed information on lipoprotein particles. Among the analytical techniques for advanced lipoprotein testing currently used the following may be mentioned:

- Density Gradient Ultracentrifugation, which allows measuring the relative cholesterol distribution for different lipoprotein subclasses (K.R. Kulkarni et al., Quantification of cholesterol in all lipoprotein classes by the VAP-II method, Journal of Lipid Research 35 (1994) 159-168). However, it does not provide either the concentration of triglycerides, or the numbers and sizes of the lipoprotein particles.

- Gradient Gel Electrophoresis, which can fractionate LDL and HDL subclasses directly from plasma according to their size (G.R. Warnick et al., Polyacrylamide gradient gel electrophoresis of lipoprotein subclasses, Clinics in Laboratory Medicine 26 (2006) 803). This method requires custom-made gels and strict attention to laboratory quality control since small variations in gel quality and laboratory conditions may affect accuracy.

- High Performance Liquid Chromatography measures the cholesterol and triglyceride content as well as the size of the major lipoproteins and their subclasses (M. Okazaki et al., Component analysis of HPLC profiles of unique lipoprotein subclass cholesterols for detection of coronary artery disease, Clinical Chemistry 52 (2006) 2049-2053).

- Ion Mobility Analysis relies on differences in electrophoretic mobility of gas-phased lipoprotein particles and allows measuring the size and concentration of some lipoprotein subclasses (M.P. Caulfield et al., Direct determination of lipoprotein particle sizes and concentrations by ion mobility analysis, Clinical Chemistry 54 (2008) 1307-1316).

- $^1$H-NMR Spectroscopy for quantifying lipoprotein subclasses based on sophisticated line-shape fitting techniques (M. Ala-Korpela et al., 1H NMR-based absolute quantification of human lipoproteins and their lipid contents directly from plasma, Journal of Lipid Research (1994) 2292-2304). However, said techniques require the use of a library of spectra previously characterized and have the disadvantage of extensive lipoprotein signal overlap in the analysis of the spectra of plasma.

- Diffusion-Ordered NMR Spectroscopy of lipoprotein fractions, which uses the methyl peak of isolated lipoproteins to calculate the diffusion coefficient of the lipoproteins and estimate their size from this value (R. Mallol et al., Particle size measurement of lipoprotein fractions using diffusion ordered NMR spectroscopy, Analytical and Bioanalytical Chemistry 402 (2012) 2407-2415). However, this method requires a previous step of ultracentrifugation of the sample in order to obtain the different lipoprotein fractions and cannot be used directly in a blood serum or blood plasma sample.

Analytical methods which physically separate the different lipoprotein fractions and subclasses, such as ultracentrifugation, are laborious and time-consuming. Moreover, samples suffer a high degree of manipulation and they might remain at 4°C for days.

Also, ALT methods are not yet ready for routine clinical use, some of their limitations being the lack of standardization and the varying approaches. Thus, there is a need for a method which allows reliable characterization of different lipoprotein fractions and subclasses directly from a blood serum or a blood plasma sample, using a single analysis and without the processing or destruction of the sample. This will be useful for developing and monitoring diet and drug therapies and getting insight into the pathophysiology of cardiovascular diseases.

Mallol R., et al., Anal Bioanal Chem 2012; 402: 2407-2415 discloses particle size measurement of lipoprotein fractions using diffusion-ordered NMR spectroscopy.

Mallol R., et al., Progr. Nucl. Mag. Res. Spectr. 2012; 70: 1-24 discloses a human serum/plasma lipoprotein analysis by NMR and its application to the study of diabetic dyslipidemia.

Stahlman M., et al., BBA - Mol. Cell Biol. Lipids. 2013; 1831: 1609-1617 discloses the association of dyslipidemia with marked alterations in the HDL lipidome in type 2 diabetic subjects in the DIWA cohort and the impact on small HSL particles.

Document WO 93/03450 A1 discloses a method and an apparatus for analysing blood plasma or serum to determine the concentrations of its lipoprotein constituents, obtaining the NMR chemical shift spectrum of a sample.

Document WO 2005043171 A1 discloses a method, a computer program product and an apparatus to determine a subject's risk of having or developing CHD using a mathematical model of in vivo concentration of LDL particles.

Document WO 2005119285 A1 discloses a process for the determination of the concentration distribution and size distribution of lipoprotein classes in body fluids, e.g. blood. For this purpose, NMR spectra of a sample to be analysed are measured by magnetic field gradient intensities and temperatures under different conditions.

## SUMMARY OF THE INVENTION

The present invention overcomes the above problems by the provision of a method according to claim 1. The dependent claims define preferred embodiments of the invention.

Currently, LDL and HDL cholesterol are two factors for cardiovascular risk that are routinely used to assess the cardiovascular risk of an individual. However, a high percentage of individuals suffering a cardiovascular event have normal levels of LDL cholesterol. Patients with metabolic disorders such as diabetes tend to have LDL lipoproteins which are smaller, poorer in cholesterol content and more atherogenic. This smaller size is associated with a greater number of particles, thus resulting in a cholesterol concentration similar to that of a pattern with a smaller concentration of larger less atherogenic particles. That is the reason why there is interest in determining the size and number of LDL lipoprotein particles, beyond their lipid load, to assess cardiovascular risk of a patient. Moreover, there are studies which indicate that the size and number of HDL particles are better predictors of cardiovascular risk than HDL cholesterol. The present invention allows exhaustive characterization of lipoprotein particles, providing in a fast and reliable way the size and number of particles of the main lipoproteins fractions and subclasses without physical separation of the different lipoprotein fractions and subclasses in the sample.

The method according to the invention comprises the following steps:

- obtaining a 2D diffusion-ordered $^1$H NMR spectrum of a sample;
- performing a surface fitting of a portion of the spectrum corresponding to the methyl signal using a plurality of model functions, each model function corresponding to a given particle size associated to a lipoprotein fraction and subclass and including at least one model parameter to be estimated during the fitting, the estimated model parameters being the set of model parameters for which the difference between the NMR signal and the model signal built as a linear combination of the model functions is minimized,

wherein each model function is a triplet of lorentzian functions having the form:

$$Triplet_j = Lorentzian(h_{1j}, f_j - f_{0j}, w_j, D_j) + Lorentzian(h_{2j}, f_j, w_j, D_j)$$

$$+ Lorentzian(h_{3j}, f_j + f_{0j}, w_j, D_j),$$

where $h_{ij}$(au), $f_j$(ppm), $w_j$(ppm), and $D_j$(cm$^2$s$^{-1}$) are the intensities, chemical shift, width, and diffusion coefficient, respectively, associated to a lipoprotein particle size $j$.

The side lorentzian functions are equally spaced in the chemical shift axis relative to the central lorentzian function, namely in an amount $f_0$.

The model parameters to be determined for a lipoprotein particle size are one or several from: $f$, $f_0$, $h_1$, $h_2$, $h_3$, $w$ and $D$.

Advantageously, the use of triplets of lorentzians as the model functions associated to each lipoprotein particle size results in a more accurate fitting of the NMR signal, when compared to other methods.

The sample is preferably a blood plasma sample or a blood serum sample, but samples of other biological fluids may be also used, such as cerebrospinal fluid, encephalorachidian fluid or amniotic fluid.

Thus, the NMR methyl signal is decomposed as a number of model functions, each model function corresponding to a lipoprotein particle size. Each model function is thus associated with a given lipoprotein fraction and subclass according to its lipoprotein particle size. Not all the model functions included in the fitting will necessarily contribute to the model signal built, since not all the lipoprotein particle sizes included in the fitting are necessarily present in the sample.

According to the method of the invention, the lipoproteins present in the sample are identified and characterized by determining the intensity, chemical shift, width, and diffusion coefficient of the associated lipoprotein signals. For further characterization of the lipoproteins present in the sample, additional lipoprotein parameters may be determined in subsequent steps based on the model functions and on the estimated model parameters.

Preferably, the lorentzian functions of the triplet associated to each lipoprotein particle size $j$ have the form

$$Lorentzian_j\left(h_j, f_j, w_j, D_j\right) = \frac{h_j}{1 + \left(\frac{f - f_j}{w_j}\right)^2} e^{-k \cdot D_j \cdot G^2}$$

where k is the Boltzmann constant and G is the gradient strength applied (Gauss cm$^{-1}$). The first quotient part of the lorentzian corresponds to a 1D lorentzian, whereas the exponential part includes the attenuation effect by means of the diffusion gradient. This preferred form of the lorentzian functions is applicable to each embodiment of the invention.

In a preferred embodiment, the method comprises identifying the lipoproteins present in the sample as those associated to the model functions having a nonzero contribution to the theoretical model signal resulting from the fitting and determining at least one and preferably all of: average size of lipoprotein particle classes, average size of lipoprotein particle subclasses, class and/or subclass lipoprotein particle concentration, lipid concentration of at least one lipoprotein particle class and/or lipid concentration of at least one lipoprotein particle subclass.

In a preferred embodiment, for the model function associated to each lipoprotein particle size $j$ the intensities of the side lorentzian functions are made proportional to the intensity of the central lorentzian function:

$$h_{1j} = \alpha_j \cdot h_{2j}, \text{with } \frac{1}{4} \leq \alpha_j \leq \frac{3}{4},$$

and

$$h_{3j} = \beta_j \cdot h_{2j}, \text{with } \frac{1}{4} \leq \beta_j \leq \frac{3}{4}.$$

In an embodiment, the proportionality factor $\alpha_j$ is made the same for all lipoprotein particle sizes and/or the proportionality factor $\beta_j$ is made the same for all lipoprotein particle sizes.

In a preferred embodiment, the intensities of the side lorentzian functions are taken to be equal, i.e. $h_{1j} = h_{3j}$.

In a preferred embodiment, $h_1 = \frac{h_2}{2}$, i.e. the intensities of the side lorentzian functions are approximately half the intensity of the central lorentzian function, and/or

$f_0$ = 0.01 ppm, i.e. the side lorentzian functions are spaced approximately 0.01 ppm in the chemical shift axis relative to the central lorentzian function.

In a preferred embodiment, the number of model functions used is greater than or equal to 9, each model function corresponding to a lipoprotein particle size.

In a preferred embodiment, the lipoprotein particle sizes used in the fitting are defined based on experimental results obtained for example by NMR, HPLC, Gradient Gel Eletrophoresis, or Atomic Force Microscope. The lipoprotein particle sizes used in the method may be selected based on any other technique.

Preferably, lipoprotein particle sizes corresponding to several lipoprotein fractions and/or subclasses are used in the method.

The surface fitting may be performed taking all the model parameters as free parameters to be determined during the fitting. However, in a preferred embodiment, the surface fitting is performed fixing a number of model parameters and using at least one other model parameter as a free parameter to be determined. More preferably, at least the signal intensity of the central lorentzian ($h_2$) is used as a free parameter and at least one of the chemical shift, width, and diffusion coefficient are fixed.

Where the number of model parameters to be estimated in the surface fitting is high, multiple solutions for the fitting appear, many of them having no biological meaning. Advantageously, fixing parameters by establishing relations between pairs of model parameters allows reducing the dimensionality of the problem and avoids the appearance of solutions with no biological relevance.

The shift of the side lorentzian functions relative to the central lorentzian function may be estimated during the surface fitting or may be taken as a given value. Similarly, the intensities of the side lorentzian functions may be estimated during the surface fitting or may be taken to be related to the intensity of the central function, preferably to be half the intensity of the central lorentzian function.

In a preferred embodiment, the fixed model parameters used in the surface fitting are determined based on the lipoprotein particle size and on regression models, the regression models relating pairs of fixed model parameters and/or a model parameter and the lipoprotein particle size.

In a preferred embodiment, the regression models used for the fixed parameters are obtained from the deconvolution of the methyl signal of a plurality of NMR spectra using a plurality of model lorentzian functions with the intensity, chemical shift, width, and diffusion coefficient being free model parameters estimated to minimize the difference between the NMR methyl signal and the model signal built as a linear combination of the model functions, the regression models respectively relating at least (i) the chemical shift and the lipoprotein particle size, and/or (ii) the width and the lipoprotein particle size.

In a preferred embodiment, the regression models relating pairs of model parameters are built according to the following steps:

- obtaining a 2D diffusion-ordered ${}^{1}$H NMR spectrum for a plurality of samples;
- for each sample, performing a surface fitting of a portion of the spectrum corresponding to the methyl signal using a plurality of model functions, each model function being dependent on the model parameters to be fixed, wherein all the model parameters to be fixed are estimated during the surface fitting as the set of model parameters for which the difference between the NMR signal and the model signal built as a linear combination of the model functions is minimized, and
- using the model parameters estimated in the previous step to build regression models relating pairs of model parameters.

Preferably, the regression models respectively relate at least (i) the chemical shift and the lipoprotein particle size, and/or (ii) the width and the lipoprotein particle size.

The plurality of samples used to build the regression models includes different lipids profiles and the number of samples considered is sufficiently high to be statistically meaningful. Preferably, the number of samples is equal to or greater than 100 and includes samples taken from healthy subjects and samples corresponding to different profiles of atherogenic dyslipidaemia. More preferably the number of samples is equal to or greater than 100 and includes samples taken from healthy subjects and, a percentage of at least 9% of the samples corresponding to individuals having a profile of diabetes mellitus and at least 25% of these patients having a profile of atherogenic dyslipidaemia.

Preferably, the model functions used to build the regression models are lorentzian function triplets of the form:

$$Triplet_j = Lorentzian\left(h_{1j}, f_j - f_{0j}, w_j, D_j\right) + Lorentzian\left(h_{2j}, f_j, w_j, D_j\right)$$
$$+ Lorentzian\left(h_{3j}, f_j + f_{0j}, w_j, D_j\right),$$

In a preferred embodiment, $h_1 = h_3 = h_2/2$.

In a preferred embodiment, the triplets of lorentzian functions used to build the regression models have the form:

$$Triplet_j = Lorentzian\left(\frac{h_j}{2}, f_j - 0.01, w_j, D_j\right) + Lorentzian\left(h_j, f_j, w_j, D_j\right)$$
$$+ Lorentzian\left(\frac{h_j}{2}, f_j + 0.01, w_j, D_j\right)$$

Alternatively, the shift ($f_0$) of the side lorentzian functions relative to the central lorentzian function may be determined during the fitting performed to build the regression models. A single value of the shift ($f_0$) may be used for all the lipoprotein sizes. Similarly, the intensities of the side lorentzian functions may be estimated during the fitting performed to build the regression models.

In a preferred embodiment the diffusion coefficient of the model functions is estimated from the lipoprotein particle size by means of the Einstein Stokes equation

$$D = \frac{kT}{6\pi\eta R_H}$$

$k$ (J K$^{-1}$) being Boltzmann constant, $T$ (K) temperature, $\eta$ (Pa s) viscosity and $R_H$ (Å) the lipoprotein particle size.

In a preferred embodiment, the method of the invention further includes correcting the estimated diffusion coefficients to take into account dilution effects, using a relation between the NMR area and the diffusion coefficient obtained for several dilutions of a sample wherein the sum of the concentration of total cholesterol and triglycerides of said sample is higher than 300 mg/dL. Advantageously, using a corrected diffusion coefficient to take into account dilution effects results in a more accurate surface fitting.

In a preferred embodiment, the NMR area associated to a lipoprotein function is corrected to consider only the contribution of the lipids included in the lipoprotein particle core.

In a preferred embodiment, the corrected NMR area (A') is determined using the following expression:

$$A' = A \cdot \frac{(9 \cdot (R - s)^3)}{[(9 \cdot (R - s)^3) + 6 \cdot p \cdot (R^3 - (R - s)^3)]}$$

with A (au) and R(Å) being respectively the area and lipoprotein particle size associated to each model function, $s$(Å) being the thickness of the lipoprotein particle shell and $p$ being the ratio of apoprotein mass per unit volume (mg/ml) in the shell of the particle relative to the total mass per unit volume (mg/ml) in the particle shell (proteins, free cholesterol and phospholipids). The proteins in the shell of the lipoprotein particles are called apolipoproteins. The ratio $p$ depends on the lipoprotein fraction. For HDL $p$ is approximately 0.5. For other lipoprotein fractions p is smaller. Generally, the thickness of the lipoprotein particle core is estimated as s = 20 ± 2Å.

As used herein, the term "apolipoproteins"or "apoproteins", i.e. proteins in the shell of the lipoprotein particles, refer to proteins that bind lipids to form lipoproteins and transport lipids through the circulatory system. Apolipoproteins are amphipathic molecules that can surround lipids creating the lipoprotein particle that is itself watersoluble and can thus be carried through water-based circulation (i.e., blood). There are six classes of apolipoproteins (A-H) and several subclasses; in particular, Apo A-I (or Apo A1) is the major protein component of HDL particles wherein Apo A-II (or Apo A2) is also present in a minor concentration. Illustrative, non-limitative, methods for determination of apolipoprotein concentration include without limitation, colorimetric methods, Western blot or ELISA. If apolipoprotein concentration is determined in an isolated fraction of lipoproteins any method well-known in the art for determining protein concentrations may be used such as enzymatic methods, colorimetric methods (Biuret, Lowry, Bradford, etc.) or immunochemical techniques (Western blot, ELISA, etc.). If apolipoprotein concentration is determined in a plasma or serum sample immunochemical techniques are used in order to obtain a specific detection of apolipoproteins. Illustrative, non-limitative, immunochemical techniques for apolipoprotein detection include immunoturbidimetric techniques, immunonefelometric techniques, radial immunodiffusion, ELISA, electroimmunoanalysis, and radio immunoanalysis.

The correction of the area is more important as the lipoprotein particle size decreases, i.e. the correction is greater for HDL particles than for LDL or VLDL particles, due to the smaller size of HDL particles.

In a preferred embodiment, the method additionally comprises determining at least one lipoprotein parameter selected from: average particle size of lipoprotein fractions, average particle size of lipoprotein subclasses, fraction lipoprotein particle concentration, subclass lipoprotein particle concentration and lipid concentration of at least one lipoprotein particle subclass and/or lipid concentration of at least one lipoprotein particle fraction.

In a preferred embodiment, the average size of a lipoprotein particle fraction is determined as:

$$Size\ (\text{Å}) = \frac{\sum_{j=1}^{n} R_j \cdot PN_j}{\sum_{j=1}^{n} PN_j},$$

n being the number of lipoprotein particle subclasses included in the lipoprotein particle fraction, $R$(Å) being the lipoprotein particle size and $PN_j$ being the particle number for said lipoprotein particle size.

In a preferred embodiment, the particle number *(PN)* for a lipoprotein is determined as:

$$PN_j \propto \frac{A_j}{R_j^3}$$

with A(au) and $R$(Å) being respectively the area and lipoprotein particle size associated to a model function $j$. Throughout the document the size of the lipoprotein particle will be understood as the lipoprotein particle radius in Angstroms. The number of lipoprotein particles of a specific size is proportional to the ratio of the area associated to the model function corresponding to said lipoprotein between the volume associated to said lipoprotein, the proportionality factor being a calibration parameter of the equipment used. Preferably, in the determination of the particle number the area associated to a lipoprotein function is the area A' corrected to consider only the contribution of the lipids included in the lipoprotein particle core, according to an embodiment of the invention.

The average size of a lipoprotein particle fraction, as used herein, refers to the average size of the radius of the lipoproteins forming part of a particular lipoprotein particle fraction.

In a preferred embodiment, the lipoprotein particle concentration of a lipoprotein particle fraction is calculated by dividing the lipid volumes by the lipoprotein particle volumes. Lipid volumes are determined by using common conversion factors to convert concentration units into volume units. Total lipoprotein particle concentrations of each main lipoprotein particle fraction are obtained by summing the concentrations of the corresponding lipoprotein particle subclasses.

In a preferred embodiment, the method comprises determining the lipid concentration of at least one lipoprotein particle fraction and/or at least one lipoprotein particle subclass. More preferably, the determination of the lipid concentration is performed using regression models. In a preferred embodiment, the regression models are calibrated with lipid concentrations measured in lipoprotein fractions obtained by ultracentrifugation in the following regions: from 5.4 to 5.15 ppm, from 3.28 to 3.14 ppm, from 2.15 to 1.85 ppm, from 1.45 to 1 ppm and from 1 to 0.7 ppm. Other methods may be used for calibration, such as ELISA, chromatography, NMR or enzymatic methods.

The determination of the lipid concentration of a lipoprotein particle fraction or subclass includes the determination of at least one of a lipid selected from triacylglycerols, cholesteryl esters, free cholesterol and phospholipids.

All the features described in this specification (including the claims, description and drawings) and/or all the steps of the described method can be combined in any combination, with the exception of combinations of such mutually exclusive features and/or steps.

## BRIEF DESCRIPTION OF DRAWINGS

To better understand the invention, its objects and advantages, the following figures are attached to the specification in which the following is depicted:

Figure 1 shows the deconvolution of a methyl signal according to the method of the invention, where the model functions used in the fitting are shown in figure 1A and the model functions grouped according to their lipoprotein fractions are shown in figure IB.

Figure 2 shows two regression models, respectively relating (a) size and chemical shift and (b) width and chemical shift.

Figure 3 shows the relation between the NMR area of the methyl signal and the diffusion coefficient.

Figure 4 shows the regions of the spectrum used in an embodiment of the method of the invention to calibrate the regression models for the lipid determination.

Figure 5 shows the deconvolution of the methyl signal of three samples.

## DETAILED DESCRIPTION OF THE INVENTION

In the *in vitro* method for the characterization of lipoprotein particles according to the invention, the samples are analysed by 2D diffusion-ordered $^1$H NMR spectroscopy.

Once the 2D diffusion-ordered $^1$H NMR spectrum has been obtained, a surface fitting is performed of the portion of the spectrum corresponding to the methyl signal. A plurality of model functions is used, each model function corresponding to a lipoprotein having a specific lipoprotein particle size and including a plurality of model parameters to be estimated during the fitting. The model function associated to each specific lipoprotein particle size is a triplet of lorentzian functions having the form:

$$Triplet = Lorentzian(h_1, f - f_0, w, D) + Lorentzian(h_2, f, w, D)$$
$$+ Lorentzian(h_3, f + f_0, w, D),$$

where $h$(au), $f$(ppm), $w$(ppm), and $D$(cm$^2$s$^{-1}$) are the model parameters to be determined and are respectively the intensity, chemical shift, width, and diffusion coefficient associated to a lipoprotein signal.

The model parameters are estimated during the fitting as the set of model parameters for which the difference between the experimental NMR signal and the model signal built as a linear combination of the model functions is minimized.

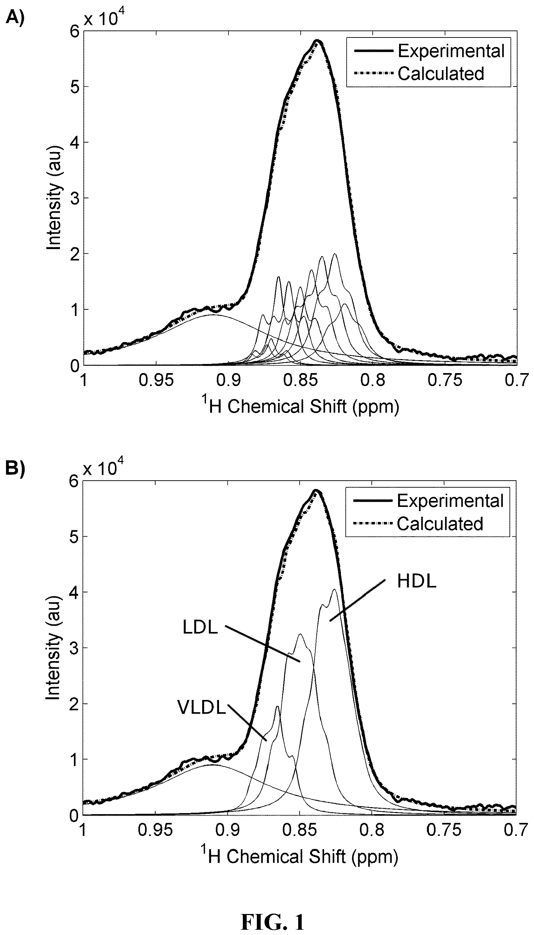

Figure 1 shows the part of a spectrum corresponding to the methyl signal of a serum sample for a single gradient of a 2D diffusion-ordered $^1$H NMR experiment and its fitting. In this case the fitting for a single gradient has been depicted for increased clarity, even if a surface fitting is performed for a plurality of diffusion gradients. Figure 1A shows in thin trace a plurality of model functions used to fit an experimental NMR spectrum, which is also shown in thick trace. The theoretical model signal built from the fitting with model functions is depicted in dot line. Each model function can be associated to a given lipoprotein particle fraction according to its lipoprotein particle size. In Figure 1B thin lines correspond to the sum of the model signals associated to each corresponding lipoprotein fraction (in this example VLDL, LDL, HDL).

In this example, the surface fitting is performed fixing all parameters (chemical shift, width and diffusion coefficient) except the signal intensities, instead of estimating all parameters for each triplet of lorentzian functions. The following form is used for the triplet lorentzian functions:

$$Triplet = Lorentzian\left(\frac{h}{2}, f - 0.01, w, D\right) + Lorentzian(h, f, w, D)$$
$$+ Lorentzian\left(\frac{h}{2}, f + 0.01, w, D\right),$$

the two signals around the central signal being shifted 0.01 ppm relative to the central signal.

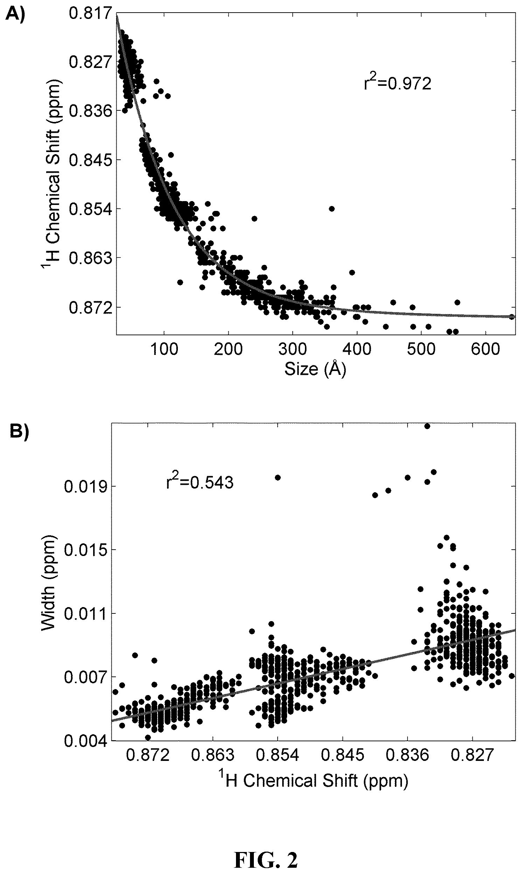

In order to fix the chemical shifts and widths associated to each model function, 300 spectra were previously deconvoluted using three triplet lorentzian functions with all their parameters free. Once the sets of parameters which best fit to the 300 experimental spectra have been obtained, they were used to fit two regression models to be used as predictors of the fixed parameters, a first regression model relating chemical shift and size (Figure 2a) and the second regression model relating width and chemical shift (Figure 2b). Figures 2a and 2b respectively show the chemical shift plotted as a function of size and the width plotted as a function of chemical shift for the estimated parameters, from which corresponding relations relating both pair of parameters can be determined. Thus, at a fixed lipoprotein particle size, its NMR chemical shift and width can be estimated using the built regression models.

A third regression model may be built to relate the diffusion coefficient with another parameter. Alternatively, the diffusion coefficient may be obtained from the lipoprotein particle size using the Einstein Stokes equation:

$$D = \frac{kT}{6\pi\eta R_H}$$

Preferably, the lipoprotein particle sizes are selected based on experiments and corresponding to a number of different lipoprotein particle subclasses, which allows a more complete and reliable lipoprotein profile to be achieved.

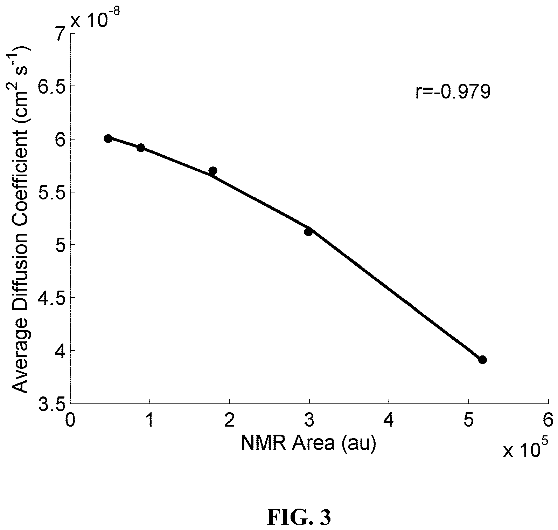

Preferably, the determined diffusion coefficients are corrected in order to take into account possible dilution effects. To study the degree of correction needed, in a preferred embodiment of the invention a serum sample having high triglycerides levels (16 mmol/L) and various dilutions of the same sample (1:2, 1:4, 1:8 and 1:16) are analyzed (Figure 3). Then, the relation between NMR area and diffusion coefficient is used to predict the correction needed for each NMR signal:

$$D_0 = \frac{D}{1 - k_{S1} \cdot A - k_{S2} \cdot A}$$

where $D_0$ is the average diffusion coefficient under dilution conditions, $D$ and A(au) are the average diffusion coefficient and the total area of the methyl peak, respectively, and $k_{S1}$, $k_{S2}$ are the regression coefficients.

To determine the average diffusion coefficient a number of spectra is obtained for each sample, each spectrum being obtained under a different gradient strength. The area under the methyl curve plotted as a function of the gradient decays exponentially with increasing gradients. The average diffusion coefficient is thus proportional to the slope of the line obtained when the logarithm of the signal attenuation $A/A_0$ is plotted versus the square of the gradient:

$$log(A/A_0) = -kDG^2$$

where $A$ is the methyl signal area, $A_0$ is the methyl signal area with zero gradient, $k$ is a constant parameter and $G$ is the gradient strength.

With the selected lipoprotein particle sizes and the chemical shift, width and diffusion coefficient determined for each lipoprotein particle size, the signal intensity is determined for each model function in order to minimize the difference between the experimental NMR signal and the model signal built from the plurality of model signals considered.

The fitting may be made by minimization of the normalized root mean squared errors (NRMSE) using the following equation:

$$NRMSE\ (\%) = \frac{\sqrt{\frac{\sum_i^n \sum_j^m (S_{exp} - S_{est})^2}{n \cdot m}}}{max(S_{exp}) - min(S_{exp})} \cdot 100$$

where $S_{exp}$ and $S_{est}$ are the experimental and estimated surfaces, respectively, $n$ is the number of data points considered and $m$ the number of gradients used.

Once the model parameters have been determined, particle-weighted lipoprotein sizes can be obtained by dividing the NMR area associated to each model function by their associated volume:

$$PN_j \propto \frac{A_j}{R_j^3}$$

where $A_j$, $R_j^3$ and $PN_j$ are the area (au), volume (Å³) and particle number (au/Å³) of a given lipoprotein particle $j$. The proportionality factor relating the particle number with the ratio between area and volume can be easily obtained by known calibration standards, which directly relate the NMR area and the lipid concentration.

Then a mean particle size can be obtained for each lipoprotein particle fraction by multiplying the NMR lipoprotein particle sizes by their fractional particle concentration relative to the total particle concentration of a given fraction:

$$Z\ (\text{Å}) = \frac{\sum_{j=1}^n R_j \cdot PN_j}{\sum_{j=1}^n PN_j}$$

where Z corresponds to mean lipoprotein particle size of a given lipoprotein particle fraction.

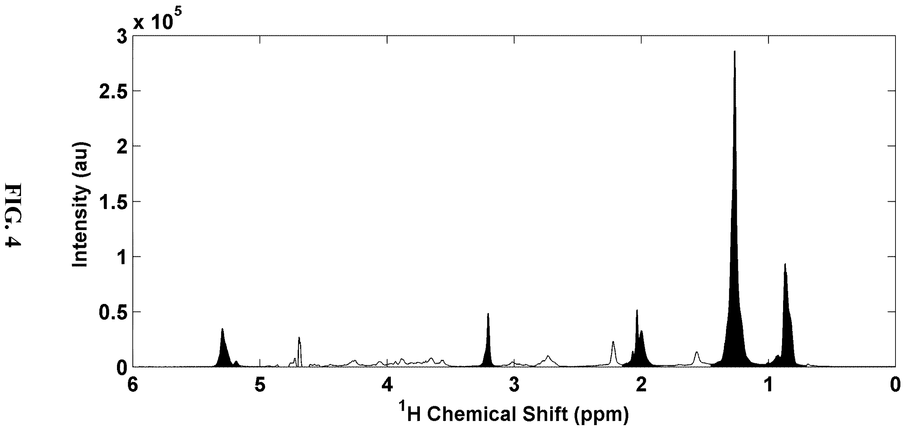

PLS regression models were calibrated to predict the cholesterol and triglyceride concentration of the main lipoprotein particle fractions (VLDL, LDL, and HDL). Regions from 5.4 to 5.15 ppm, from 3.28 to 3.14 ppm, from 2.15 to 1.85 ppm, from 1.45 to 1 ppm and from 1 to 0.7 ppm (shown in Figure 4) were used as X-Block and the cholesterol and triglyceride concentrations were used as Y-Block. Both blocks were mean-centered. The optimum number of latent variables (LV) and the validation performance of the PLS models, were assessed using venetian blinds cross-validation splitting the data 10 times. Coefficients of determination between the predicted and reference concentrations ranged from 0.79 to 0.98 in the calibration step. The coefficients of determination of the validation step ranged from 0.81 to 0.98.

The determination of the lipid concentration of a lipoprotein particle fraction or subclass includes the determination of at least one of a lipid selected from triacylglycerols, cholesteryl esters, free cholesterol and phospholipids.

A triglyceride (or triacylglycerol) is an ester derived from glycerol and three fatty acids which can be found in a lipoprotein particle. Illustrative, non-limitative, fatty acids that can be found in lipoprotein triglycerides are palmitic acid, estearic acid, oleic acid, linoleic acid and araquidonic acid. Triglycerides are blood lipids that help enable the bidirectional transference of adipose fat and blood glucose from the liver. Illustrative, non-limitative, methods for determination of triglycerides concentration include enzymatic determination. Enzymatic determination of triglycerides is also possible because it is specific and sensitive. The principle of reaction is as follows: Triglycerides are hydrolyzed by a lipase in glycerol and free fatty acids. In the presence of glycerol kinase, glycerol is phosphorylated to glycerol-3-phosphate which is then oxidized with a glycerol phosphate oxidase with formation of hydrogen peroxide. In the presence of peroxidase, 4-chlorophenol and 4-aminoantipyrine with hydrogen peroxide yield a red-colored product, quinonimine. The staining intensity is directly proportional to the sample concentration of triglycerides.

Cholesterol is an amphipatic lipid. A cholesteryl ester is an ester of cholesterol wherein the ester bond is formed between the carboxylate group of a fatty acid and the hydroxyl group of cholesterol. Illustrative, non-limitative, examples of cholesteryl esters present in a lipoprotein particle are cholesterylpalmitate, cholesteryl stearate, cholesteryloleate, cholesteryllinoleate and cholesterylaraquidonate (Skipski, V.P. In: Blood Lipids and Lipoproteins. Quantitation, Composition and Metabolism. pp.471-483 (ed. G.J. Nelson, Wiley-Interscience, New York) (1972)). Cholesteryl esters have a lower solubility in water than cholesterol and are more hydrophobic. Numerous methods are available for determination of cholesterol concentration, e.g., gravimetric, nephelometric, turbidimetric, or photometric methods, among others. Commercially available kits for quantitative colorimetric/fluorimetric cholesterol and cholesteryl esters determination may be used. Usually, the concentrations of total and free cholesterol (esterified cholesterol being previously precipitated by, for example, digitonin) are determined, whereas the concentration of cholesteryl esters (esterified cholesterol) is calculated from the difference between these two concentrations. Enzymatic determination of cholesterol concentration is specific and sensitive. The principle of reaction is as follows: Cholesterol esterase catalyzes hydrolysis of cholesteryl esters to free cholesterol and free fatty acids. In the presence of cholesterol oxidase, cholesterol is oxidized to $\delta$-4-cholestanetriol to form hydrogen peroxide. In the presence of peroxidase, phenol and 4-aminoantipyrine with hydrogen peroxide yield a red-colored product, quinonimine. The staining intensity is directly proportional to the sample concentration of total cholesterol.

Phospholipids are a class of lipids that are present in the shell of lipoprotein particles and that are a major component of all cell membranes as they can form lipid bilayers. Most phospholipids contain a diglyceride, a phosphate group, and a simple organic molecule such as choline. The structure of the phospholipid molecule generally consists of hydrophobic tails and a hydrophilic head. Illustrative, non-limitative, examples of phospholipids present in a lipoprotein particle are phosphatidylcholine, sphingophospholipids such as sphingomyelin, phosphatidylethanolamine, phosphatidylinositol and phosphatidylserine. Illustrative, non-limitative, methods for determination of phospholipids concentration, include commercially available assay kits for a quantitative colorimetric/fluorimetric phospholipid determination. The principle of reaction is as follows: phospholipids (such as lecithin, lysolecithin and sphingomyelin) are enzymatically hydrolyzed to choline which is determined using choline oxidase and a $H_2O_2$ specific dye. The optical density of the pink colored product at 570nm or fluorescence intensity (530/585 nm) is directly proportional to the phospholipid concentration in the sample.

Example

2D diffusion-ordered $^1$H NMR spectroscopy (DOSY):

Serum samples were analysed by NMR spectroscopy, recording 1H NMR spectra on a BrukerAvance III spectrometer at 310 K. The double stimulated echo (DSTE) pulse program was used with bipolar gradient pulses and a longitudinal eddy current delay (LED). The relaxation delay was 2 s, the free induction decays were collected into 64K complex data points and 32 scans were acquired on each sample. The gradient pulse strength was increased from 5 to 95% of the maximum strength of 53.5 Gauss cm$^{-1}$ in 32 steps, where the squared gradient pulse strength was linearly distributed.

Surface fitting:

In the present example the lipoprotein NMR signals were modeled as triplets of lorentzian functions, with the two signals around the central signal shifted 0.01 ppm:

$$Triplet = Lorentzian\left(\frac{h}{2}, f - 0.01, w, D\right) + Lorentzian(h, f, w, D)$$

$$+ Lorentzian\left(\frac{h}{2}, f + 0.01, w, D\right)$$

where $h$ (au), $f$ (ppm), $w$ (ppm), and $D$ (cm$^2$ s$^{-1}$) are the intensity, chemical shift, width, and diffusion coefficient of a given lipoprotein signal, each lipoprotein signal corresponding to a lipoprotein particle size. 9 lipoprotein particle sizes and consequently also 9 model functions were used based on lipoprotein particle sizes obtained by HPLC.

The 9 model functions were associated with a given lipoprotein particle fraction (VLDL, LDL or HDL) according to their NMR size. Thus, functions F1-F3 were associated to VLDL, functions F4-F6 to LDL, and functions F7-F9 to HDL (Table 1). Main lipoprotein particle fractions were defined as VLDL (193-409 Å), LDL (74-133 Å) and HDL (30-55 Å).

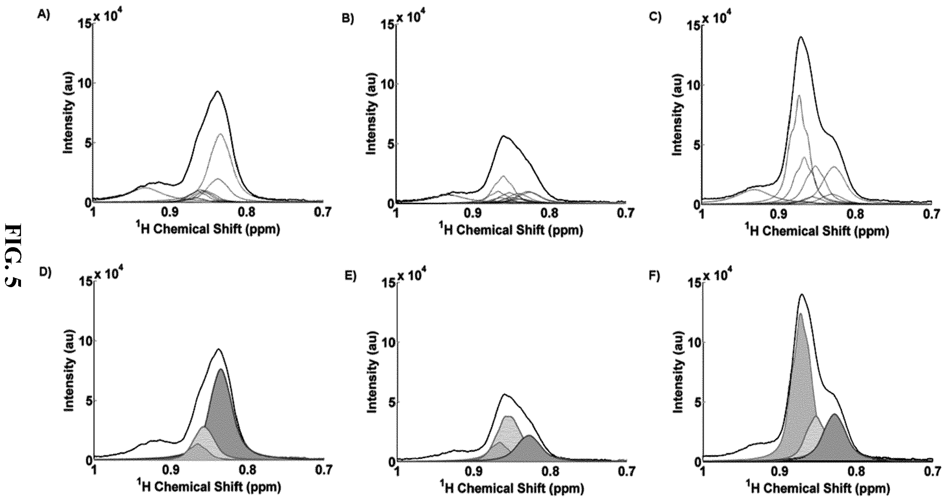

The surface fitting of the methyl signal was performed using 9 model functions with all parameters fixed (chemical shift, width and diffusion coefficient) except the signal intensity ($h$). The fitting of each sample elapsed $29.47\pm4.42$ seconds on average. The methyl signal was thus decomposed into individual lipoprotein signals to obtain the contribution of 9 lipoproteins with fixed NMR size. Figures 5A-C show the results of the surface fitting for three subjects, which are also summarized in Table 1. It must be noted that even using 9 model functions to fit the spectra, not all of them are used to find the final solution. Figures 5D-F show the grouping of model functions according to their associated lipoprotein particle main fraction. Subject 1 shows a prominent HDL area (shown in dark grey in Figure 5D), subject 2 has increased LDL area (shown in light grey in Figure 5E), and subject 3 is characterized by a very high VLDL area (shown in medium grey in Figure 5F).

The uniqueness of the solutions was studied by fitting each sample ten times with randomly chosen initial values of the signal intensities. Because the dynamic range in signal intensity may be very high, the following formula was used to assess the coefficient of variation (CV) for each model function and sample across ten different fittings:

$$CV = \frac{SD\ (h)}{Max(h) - Min(h)} \cdot 100\ (\%)$$

where $h$ stands for signal intensity of a given model function. Maximum (Max), minimum (Min) and standard deviations (SD) values where assessed from the ten fittings in each sample. As a result, unique solutions were obtained for all samples after 10 runs.

In this case the coefficient of variation (CV) is determined for the signal intensity of the central lorentzian, since the other model parameters have been fixed during the fitting. The above expression for the calculation of the coefficient of variation may be generalized for the case where more than one free model parameters is used in the fitting.

Normalized root mean squared errors (NRMSE) of the fittings were calculated using the following equation

$$NRMSE\ (\%) = \frac{\sqrt{\dfrac{\Sigma_i^n \Sigma_j^m (S_{exp} - S_{est})^2}{n \cdot m}}}{max(S_{exp}) - min(S_{exp})} \cdot 100$$

where $S_{exp}$ and $S_{est}$ are the experimental and estimated surfaces, respectively, $n$ is the number of data points considered in interval length (0.7-1 ppm) and $m$ the number of gradients used. In this example, $n$ and m had the same value for each sample. The average NRMSE obtained was of less than 1.5%.

To obtain particle-weighted lipoprotein sizes, we first divided each NMR area by their associated volume:

$$PN_j \propto \frac{A_j}{R_j^3}$$

where $A_j$, $R_j^3$ and $PN_j$ are the area (au), volume (Å$^3$) and particle number (au/Å$^3$) of a given lipoprotein particle $j$.

Then, the mean particle size for each lipoprotein particle fraction was obtained multiplying the lipoprotein particle sizes by their fractional particle concentration relative to the total particle concentration of a given particle fraction:

$$VLDL\ Size\ (\text{Å}) = \frac{\sum_{j=1}^{n} R_j \cdot PN_j}{\sum_{j=1}^{n} PN_j}, j = 1, \dots, 3$$

$$LDL\ Size\ (\text{Å}) = \frac{\sum_{j=1}^{n} R_j \cdot PN_j}{\sum_{j=1}^{n} PN_j}, j = 4, \dots, 6$$

$$HDL\ Size\ (\text{Å}) = \frac{\sum_{j=1}^{n} R_j \cdot PN_j}{\sum_{j=1}^{n} PN_j}, j = 7, \dots, 9$$

Particle concentrations of each lipoprotein particle subclass were calculated by dividing the lipid volumes by the particle volumes. Lipid volumes were determined by using common conversion factors to convert concentration units into volume units. Total particle concentrations of each main particle fraction were obtained by summing the concentrations of the corresponding particle subclasses.

Lipid concentration was determined using PLS models calibrated in the regions shown in Figure 4, namely: from 5.4 to 5.15 ppm, from 3.28 to 3.14 ppm, from 2.15 to 1.85 ppm, from 1.45 to 1 ppm and from 1 to 0.7 ppm. The reference lipids were obtained by sequential ultracentrifugation and correspond to cholesterol and triglycerides for three lipoprotein particle fractions (VLDL, LDL and HDL).

The method of the invention allows to obtain an advanced lipoprotein profile, as shown in Table 1. The ALT reports for three representative subjects from the whole group are summarized in Table 1 for illustration purposes. Subject 1 was normolipidemic, subject 2 presented high LDL cholesterol levels (hypercholesterolemic) and subject 3 presented high triglycerides and low HDL cholesterol levels (atherogenic dyslipidemia). The method of the invention provides total cholesterol (C), triglycerides (TG) and particle concentration (P) for the main lipoprotein fractions and their subclasses. Additionally, the method of the invention provides the size of the main lipoprotein fractions. Subject 1 showed normal lipid levels (defined as VLDL-TG<150 mg/dL, LDL-C<160 mg/dL, and HDL-C>40 mg/dL), subject 2 showed elevated LDL-C and normal VLDL-TG and HDL-C levels, and subject 3 showed elevated VLDL-TG, decreased HDL-C and normal LDL-C levels. It should also be pointed out that subject 3 had increased LDL-P despite normal LDL-C levels and that its small LDL-P concentration was higher than in subject 2. Thus, subjects with elevated triglycerides levels are associated with increased LDL-P values because an increase in triglyceride concentration leads to the formation of greater concentrations of smaller LDL particles.

Advanced lipoprotein tests have shown statistical associations between these lipoprotein parameters and the risk for cardiovascular disease (Sniderman, A., and P. O. Kwiterovich. 2013. Update on the detection and treatment of atherogenic low-density lipoproteins. Current opinion in endocrinology, diabetes, and obesity 20: 140-147). For example, in a study, the number of HDL particles (HDL-P), but not the high-density lipoprotein cholesterol (HDL-C) concentrations, were independently associated with carotid intima-media thickness, after adjusting for covariates (Mackey, R. H., P. Greenland, D. C. Goff, Jr., D. Lloyd-Jones, C. T. Sibley, and S. Mora. 2012. High-density lipoprotein cholesterol and particle concentrations, carotid atherosclerosis, and coronary events: MESA (multi-ethnic study of atherosclerosis). J Am CollCardiol 60: 508-516). Finally, the use of lipoprotein particle subclasses improved NMR-derived risk stratification for subclinical atherosclerosis compared with conventional lipid measures in the prediction models with risk factors of the Framingham risk score (Wurtz, P., J. R. Raiko, C. G. Magnussen, P. Soininen, A. J. Kangas, T. Tynkkynen, R. Thomson, R. Laatikainen, M. J. Savolainen, J. Laurikka, P. Kuukasjarvi, M. Tarkka, P. J. Karhunen, A. Jula, J. S. Viikari, M. Kahonen, T. Lehtimaki, M. Juonala, M. Ala-Korpela, and O. T. Raitakari. 2012. High-throughput quantification of circulating metabolites improves prediction of subclinical atherosclerosis. European Heart Journal 33: 2307-2316).

**Table 1.** Summary of lipoprotein parameters obtained using the method of the invention

|  |  | Subject 1 | Subject 2 | Subject 3 |
|---|---|---|---|---|
| Lipids (mg/dL) | VLDL-TG | 13.7 | 22.6 | 150.3 |
|  | LDL-C | 105.4 | 161.8 | 127.0 |
|  | LDL-TG | 14.9 | 20.9 | 18.4 |

(continued)

|  |  | Subject 1 | Subject 2 | Subject 3 |
|---|---|---|---|---|
|  | HDL-C | 66.5 | 49.5 | 34.4 |
|  | HDL-TG | 7.1 | 6.6 | 11.0 |
| ParticleConcentration* | VLDL-P | 12.4 | 22.0 | 106.3 |
|  | LargeVLDL-P | 0.2 | 0.3 | 5.9 |
|  | Medium VLDL-P | 1.0 | 1.9 | 21.6 |
|  | Small VLDL-P | 11.1 | 19.7 | 78.8 |
|  | LDL-P | 980.6 | 1399.1 | 1230.4 |
|  | LargeLDL-P | 80.0 | 184.3 | 23.7 |
|  | Medium LDL-P | 212.4 | 406.8 | 310.5 |
|  | Small LDL-P | 688.2 | 808.0 | 896.1 |
|  | HDL-P | 32.2 | 27.1 | 25.9 |
|  | LargeHDL-P | 2.4 | 1.3 | 0.3 |
|  | Medium HDL-P | 10.0 | 6.8 | 3.6 |
|  | Small HDL-P | 19.8 | 19.0 | 22.0 |
| Size (nm) | VLDL | 39.0 | 38.9 | 42.2 |
|  | LDL | 19.7 | 20.1 | 19.5 |
|  | HDL | 8.2 | 8.0 | 7.8 |
| *VLDL/LDL: nmol/L; HDL: $\mu$mol/L | | | | |

## Claims

1. An *in vitro* method for the characterization of lipoproteins in a sample, comprising the following steps:

- obtaining a 2D diffusion-ordered $^1$H NMR spectrum of the sample,
- performing a surface fitting of a portion of the spectrum corresponding to the methyl signal using a plurality of model functions, each model function corresponding to a given particle size associated to a lipoprotein fraction and subclass and including at least one model parameter to be estimated during the fitting, the estimated model parameters being the set of model parameters for which the difference between the NMR signal and the model signal built as a linear combination of the model functions is minimized, and
- identifying the lipoproteins present in the sample as those associated to the model functions contributing to the theoretical model signal resulting from the fitting, wherein each model function is a triplet of lorentzian functions having the form:

$$Triplet = Lorentzian(h_1, f - f_0, w, D) + Lorentzian(h_2, f, w, D)$$
$$+ Lorentzian(h_3, f + f_0, w, D),$$

where $h$(au), $f$(ppm), $w$(ppm), and $D$(cm$^2$s$^{-1}$) are, respectively, the intensity, chemical shift, width, and diffusion coefficient associated to a lipoprotein particle size, wherein the model parameters to be determined for each lipoprotein particle size are one or several from: $f$, $f_0$, $h_1$, $h_2$, $h_3$, $w$ and $D$,

wherein for each model function:

$$h_1 = \alpha \cdot h_2, \text{with } \frac{1}{4} \leq \alpha \leq \frac{3}{4},$$

and

$$h_3 = \beta \cdot h_2, \text{with } \frac{1}{4} \leq \beta \leq \frac{3}{4},$$

wherein the surface fitting is performed fixing at least one model parameter and using at least one other model parameter as a free parameter to be determined in the surface fitting,
wherein the fixed model parameters are determined based on the lipoprotein particle size and on regression models, the regression models relating pairs of fixed model parameters and/or a model parameter and the lipoprotein particle size and being built according to the following steps:

- obtaining a 2D diffusion-ordered $^1$H NMR spectrum for a plurality of samples,
- for each sample, performing a surface fitting of a portion of the spectrum corresponding to the methyl signal using a plurality of model functions, each model function being dependent on the model parameters to be fixed, wherein all the model parameters to be fixed are estimated during the surface fitting as the set of model parameters for which the difference between the NMR signal and the model signal built as a linear combination of the model functions is minimized, and
- using the model parameters estimated in the previous step to build regression models relating pairs of model parameters and/or a model parameter and the lipoprotein particle size.

2. The *in vitro* method according to claim 1, wherein the triplets of lorentzian functions have the form:

$$Triplet = Lorentzian(h_1, f - f_0, w, D) + Lorentzian(h_2, f, w, D) + Lorentzian(h_1, f + f_0, w, D)$$

3. The *in vitro* method according to any of the previous claims, wherein

$$h_1 = \frac{h_2}{2},$$

and/or

$$f_0 = 0.01 \text{ ppm}.$$

4. The *in vitro* method according to any of the previous claims, wherein the lipoprotein particle sizes are defined based on NMR, HPLC, Gradient Gel Electrophoresis or Atomic Force Microscope experiments.

5. The *in vitro* method according to any of the previous claims, wherein at least one of the chemical shifts, width, and diffusion coefficient is fixed and at least the signal intensity of the central lorentzian ($h_2$) is used as a free parameter.

6. The *in vitro* method according to any of claims 1 to 5, wherein the model functions used to build the regression models are lorentzian function triplets of the form:

$$Triplet_j = Lorentzian(h_{1j}, f_j - f_{0j}, w_j, D_j) + Lorentzian(h_{2j}, f_j, w_j, D_j) + Lorentzian(h_{3j}, f_j + f_{0j}, w_j, D_j).$$

7. The *in vitro* method according to any of the previous claims, wherein the plurality of samples used to build the regression models comprise at least 100 samples and a percentage of at least 9% of the samples corresponds to individuals having a profile of diabetes mellitus and at least 25% of these patients having a profile of atherogenic dyslipidaemia.

8. The *in vitro* method according to any of the previous claims, wherein the diffusion coefficient of the model functions is estimated from the lipoprotein particle size by means of the Einstein Stokes equation

$$D = \frac{kT}{6\pi\eta R_H}$$

with $k$ (J K$^{-1}$) being Boltzmann constant, $T$ (K) temperature, $\eta$ (Pa s) viscosity and $R_H$ (Å) the lipoprotein particle size.

9. The *in vitro* method according to any of the previous claims, the method further including correcting the estimated diffusion coefficients to take into account dilution effects, based on a relation between the NMR area and the diffusion coefficient obtained for several dilutions of a sample wherein the sum of the concentration of total cholesterol and triglycerides of said sample is higher than 300 mg/dL.

10. The *in vitro* method according to any of the previous claims, further comprising determining one or more of: average size of lipoprotein particle fractions, average size of lipoprotein particle subclasses, fraction and/or subclass lipoprotein particle concentration, lipid concentration of at least one lipoprotein particle fraction and/or lipid concentration of at least one lipoprotein particle subclass.

11. The *in vitro* method according to claim 10, wherein the average particle size of a lipoprotein particle fraction is determined as:

$$Size \ (\text{Å}) = \frac{\sum_{j=1}^{n} R_j * PN_j}{\sum_{j=1}^{n} PN_j},$$

n being the number of lipoprotein particle subclasses included in the lipoprotein particle fraction, $R$(Å) being the lipoprotein particle size and $PN_j$ being the particle number for said lipoprotein particle size, wherein the particle number ($PN$) for a lipoprotein $j$ is determined as:

$$PN_j \propto \frac{A_j}{R_j^3}$$

with A(au) being the area associated to each model function.

12. The *in vitro* method according to any of the previous claims, wherein the area associated to a lipoprotein model function is corrected to consider only the contribution of the lipids included in the lipoprotein particle core.

13. The *in vitro* method according claim 12 wherein the corrected area ($A'$) is determined using the following expression:

$$A' = A \cdot \frac{(9 \cdot (R - s)^3)}{[(9 \cdot (R - s)^3) + 6 \cdot p \cdot (R^3 - (R - s)^3)]}$$

with $A$ (au) and $R$(Å) being respectively the area and lipoprotein particle size associated to each model function, $s$(Å) being the lipoprotein particle shell thickness and p being the ratio of protein mass in the shell of the particle relative to the total mass in the particle shell.

**Patentansprüche**

1. *In-vitro*-Verfahren zum Charakterisieren von Lipoproteinen in einer Probe, umfassend die folgenden Schritte:

  - Erhalten eines 2D diffusionsgeordneten $^1$H NMR-Spektrums der Probe,
  - Durchführen einer Oberflächenanpassung eines Teils des Spektrums entsprechend den Methylsignalen unter Verwendung einer Vielzahl von Modellfunktionen, wobei jede Modellfunktion einer gegebenen Partikelgröße entspricht, die einer Lipoproteinfraktion und Unterklasse zugeordnet ist und zumindest einen Modellparameter umfasst, der während des Anpassens zu bestimmen ist, wobei die bestimmten Modellparameter ein Satz von Modellparametern sind, für die der Unterschied zwischen dem NMR-Signal und dem Modellsignal als eine lineare Kombination von Modellfunktionen minimiert werden, und

- Identifizieren der Lipoproteine, die in der Probe vorhanden sind als diejenigen, die den Modellfunktionen zugeordnet sind, beitragend zu dem theoretischen Modellsignal, das aus dem Anpassen resultiert, wobei jede Modellfunktion ein Triplet von Lorentz-Funktionen mit der folgenden Form ist:

$$Triplet = Lorentz(h_1, f - f_0, w, D) + Lorentz(h_2, f, w, D)$$
$$+ Lorentz(h_3, f + f_0, w, D),$$

wobei $h$(au), $f$(ppm), $w$(ppm) und $D$(cm$^2$s$^{-1}$) jeweils die Intensität, die chemische Verschiebung, Breite und Diffusionskoeffizient, die einer Lipoproteinpartikelgröße zugeordnet sind, sind, wobei die Modellparameter, die für jede Lipoproteinpartikelgröße zu bestimmen sind, einer oder mehrere aus den Folgenden sind: $f$, $f_0$, $h_1$, $h_2$, $h_3$, $w$ und $D$,

wobei für jede Modellfunktion:

$$h_1 = \alpha \cdot h_2, \text{ mit } \frac{1}{4} \leq \alpha \leq \frac{3}{4},$$

und

$$h_3 = \beta \cdot h_2, \text{ mit } \frac{1}{4} \leq \beta \leq \frac{3}{4},$$

wobei die Oberflächenanpassung durch Festsetzen zumindest eines Modellparameters und Verwenden zumindest eines anderen Modellparameters als einen freien Parameter, der bei der Oberflächenanpassung zu bestimmen ist, durchgeführt wird,
wobei die festgesetzten Modellparameter basierend auf der Lipoproteinpartikelgröße und auf Regressionsmodellen basierend bestimmt werden, wobei die Regressionsmodelle sich auf Paare von festgesetzten Modellparametern und/oder einem Modellparameter und der Lipoproteinpartikelgröße beziehen und gemäß den folgenden Schritten bestimmt werden:

- Erhalten eines 2D diffusionsgeordneten $^1$H NMR-Spektrums für eine Vielzahl von Proben,
- für jede Probe Durchführen einer Oberflächenanpassung für einen Teil des Spektrums entsprechend den Methylsignalen unter Verwendung einer Vielzahl von Modellfunktionen, wobei jede Modellfunktion von festzusetzenden Modellparametern abhängt, wobei alle diese Modellparameter, die festzusetzen sind, während der Oberflächenanpassung derart angenähert werden, um zu einem Satz von Modellparametern, für die der Unterschied zwischen dem NMR-Signal und dem Modellsignal, das durch eine lineare Kombination von Modellfunktionen erhalten wird, minimiert wird, und
- Verwenden der Modellparameter, die in dem vorangehenden Schritt angenähert werden, um Regressionsmodelle zu bilden, die sich auf Paare von Modellparametern und/oder einem Modellparameter und der Lipoproteinpartikelgröße beziehen.

2. *In-vitro*-Verfahren nach Anspruch 1, wobei die Triplets der Lorentz-Funktionen die folgende Form haben:

$$Triplet = Lorentz(h_1, f - f_0, w, D) + Lorentz(h_2, f, w, D)$$
$$+ Lorentz(h_1, f + f_0, w, D)$$

3. *In-vitro*-Verfahren nach einem der vorangehenden Ansprüche, wobei

$$h_1 = \frac{h_2}{2},$$

und/oder

$$f_o = 0{,}01 \text{ ppm}$$

ist.

**4.** *In-vitro*-Verfahren nach einem der vorangehenden Ansprüche, wobei die Lipoproteinpartikelgrößen basierend auf NMR, HPLC, Gradientengelelektrophorese oder Rasterkraftmikroskopexperimenten definiert werden.

**5.** *In-vitro*-Verfahren nach einem der vorangehenden Ansprüche, wobei zumindest eine der chemischen Verschiebungen, der Breite und des Diffusionskoeffizienten festgesetzt werden und zumindest die Signalintensität des zentralen Lorentz-Parameters ($h_2$) als ein freier Parameter verwendet wird.

**6.** *In-vitro*-Verfahren nach einem der Ansprüche 1 bis 5, wobei die Modellfunktionen, die zum Bilden der Regressionsmodelle verwendet werden, Lorentz-Funktionstriplets der folgenden Form sind:

$$Triplet_j = Lorentz(h_{1j}, f_j - f_{oj}, w_j, D_j) + Lorentz(h_{2j}, f_j, w_j, D_j)$$
$$+ Lorentz(h_{3j}, f_j + f_{oj}, w_j, D_j).$$

**7.** *In-vitro*-Verfahren nach einem der vorangehenden Ansprüche, wobei die Vielzahl der Proben, die zum Bilden der Regressionsmodelle verwendet werden, zumindest 100 Proben umfassen und ein Prozentsatz von zumindest 9% der Proben Individuen entsprechen, die ein Profil von Diabetes mellitus haben und zumindest 25% dieser Patienten ein Profil von atherogener Dyslipidämie haben.

**8.** *In-vitro*-Verfahren nach einem der vorangehenden Ansprüche, wobei der Diffusionskoeffizient der Modellfunktionen ausgehend von der Lipoproteinpartikelgröße unter Zuhilfenahme der Einstein-Stokes-Gleichung bestimmt wird

$$D = \frac{kT}{6\pi\eta R_H}$$

mit $k$ (J K$^{-1}$) Boltzmann-Faktor, $T$ (K) Temperatur, $\eta$ (Pa s) Viskosität und $R_H$ (Å) die Lipoproteinpartikelgröße.

**9.** *In-vitro*-Verfahren nach einem der vorangehenden Ansprüche, wobei das Verfahren ferner das Korrigieren der abgeschätzten Diffusionskoeffizienten, um Verdünnungseffekte zu berücksichtigen, umfasst, basierend auf einem Zusammenhang zwischen der NMR-Fläche und dem Diffusionskoeffizienten, der für verschiedene Verdünnungen einer Probe erhalten wird, wobei die Summe der Konzentration des gesamten Cholesterols und Triglyceride der besagten Probe höher als 300 mg/dl ist.

**10.** *In-vitro*-Verfahren nach einem der vorangehenden Ansprüche, ferner umfassend das Bestimmen einer oder mehrerer von: durchschnittliche Größe der Lipoproteinpartikelfraktionen, durchschnittliche Größe der Lipoproteinpartikelunterklassen, Fraktion und/oder Unterklasse der Lipoproteinpartikelkonzentration, Lipidkonzentration von zumindest einer Lipoproteinpartikelfraktion und/oder Lipidkonzentration von zumindest einer Lipidproteinpartikelunterklasse.

**11.** *In-vitro*-Verfahren nach Anspruch 10, wobei die durchschnittliche Partikelgröße einer Lipoproteinpartikelfraktion bestimmt wird als:

$$Größe\ (Å) = \frac{\sum_{j=1}^{n} R_j * PN_j}{\sum_{j=1}^{n} PN_j},$$

mit n gleich Anzahl der Lipoproteinpartikelunterklassen, die in der Lipoproteinpartikelfraktion enthalten sind, $R(Å)$ gleich Lipoproteinpartikelgröße und $PN_j$ gleich Partikelanzahl für besagte Lipoproteinpartikelgröße, wobei die Partikelanzahl ($PN$) für Lipoprotein $j$ bestimmt wird als:

$$PN_j \propto \frac{A_j}{R_j^3}$$

mit A(au) gleich Fläche, die der jeweiligen Modellfunktion zugeordnet ist.

**12.** *In-vitro*-Verfahren nach einem der vorangehenden Ansprüche, wobei die Fläche, die einer Lipoproteinmodellfunktion zugeordnet ist, korrigiert wird, um lediglich den Beitrag der Lipide zu berücksichtigen, die in dem Lipoproteinpartikelkern umfasst sind.

**13.** *In-vitro*-Verfahren nach Anspruch 12, wobei die korrigierte Fläche (*A'*) bestimmt wird unter Verwendung des folgenden Ausdrucks:

$$A' = A \cdot \frac{(9 \cdot (R-s)^3)}{[(9 \cdot (R-s)^3) + 6 \cdot p \cdot (R^3 - (R-s)^3)]}$$

mit *A*(au) und *R*(Å) jeweils gleich die Fläche und Lipoproteinpartikelgröße, die den jeweiligen Modellfunktionen zugeordnet sind, *s*(Å) gleich Lipoproteinpartikelschalendicke und p gleich Verhältnis der Proteinmasse in der Schale der Partikel zu dem Gesamtgewicht in der Partikelschale.

## Revendications

**1.** Procédé *in vitro* pour la caractérisation de lipoprotéines dans un échantillon, comprenant les étapes suivantes:

- obtenir un spectre RMN $^1$H d'ordre de diffusion 2D de l'échantillon,
- effectuer un réglage de surface d'une partie du spectre correspondant au signal du méthyle à l'aide d'une pluralité de fonctions modèles, chaque fonction modèle correspondant à une dimension de particule donnée associée à une fraction et une sous-classe de lipoprotéine et comprenant au moins un paramètre modèle à estimer pendant le réglage, les paramètres modèles estimés étant l'ensemble de paramètres modèles pour lequel la différence entre le signal RMN et le signal modèle construit comme combinaison linéaire des fonctions modèles est rendue minimale, et
- identifier les lipoprotéines présentes dans l'échantillon comme celles étant associées aux fonctions modèles contribuant au signal modèle théorique résultant du réglage, chaque fonction modèle étant est un triplet de fonctions lorentziennes ayant la forme :

$$Triplet = Lorentzienne(h_1, f - f_0, w, D) + Lorentzienne(h_2, f, w, D)$$
$$+ Lorentzienne(h_3, f + f_0, w, D)$$

où *h*(ua), *f*(ppm), *w*(ppm), et *D*(cm$^2$s$^{-1}$) sont, respectivement, l'intensité, le déplacement chimique, la largeur, et le coefficient de diffusion associés à une dimension de particule de lipoprotéine, les paramètres modèles à déterminer pour chaque dimension de particule de lipoprotéine étant un ou plusieurs éléments parmi : $f$, $f_0$, $h_1$, $h_2$, $h_3$, $w$ et $D$,

dans lequel, pour chaque fonction modèle :

$$h_1 = \alpha \cdot h_2, \text{ avec } \frac{1}{4} \leq \alpha \leq \frac{3}{4},$$

et

$$h_3 = \beta \cdot h_2, \text{ avec } \frac{1}{4} \leq \beta \leq \frac{3}{4},$$

dans lequel le réglage de surface est effectué en fixant au moins un paramètre modèle et en utilisant au moins un autre paramètre modèle comme paramètre libre à déterminer dans le réglage de surface,
dans lequel les paramètres modèles fixes sont déterminés sur la base de la dimension de particule de lipoprotéine et sur des modèles de régression, les modèles de régression mettant en relation des paires de paramètres modèles fixes et/ou un paramètre modèle et la dimension de particule de lipoprotéine et étant construits selon les étapes suivantes:

- obtenir un spectre RMN $^1$H d'ordre de diffusion 2D pour une pluralité d'échantillons,
- pour chaque échantillon, effectuer un réglage de surface d'une partie du spectre correspondant au signal du méthyle à l'aide d'une pluralité de fonctions modèles, chaque fonction modèle étant dépendante des paramètres modèles à fixer, tous les paramètres modèles à fixer étant estimés pendant le réglage de surface comme l'ensemble de paramètres modèles pour lesquels la différence entre le signal RMN et le signal modèle construit comme combinaison linéaire des fonctions modèles est rendue minimale, et
- utiliser les paramètres modèles estimés dans l'étape précédente pour construire des modèles de régression mettant en relation des paires de paramètres modèles et/ou un paramètre modèle et la dimension de particule de lipoprotéine.

2. Procédé *in vitro* selon la revendication 1, dans lequel les triplets de fonctions lorentziennes ont la forme suivante :

$$Triplet = Lorentzienne(h_1, f - f_0, w, D) + Lorentzienne(h_2, f, w, D)$$
$$+ Lorentzienne(h_1, f + f_0, w, D).$$

3. Procédé *in vitro* selon l'une quelconque des revendications précédentes, dans lequel

$$h_1 = \frac{h_2}{2},$$

et/ou

$$f_0 = 0,01 \text{ ppm}.$$

4. Procédé *in vitro* selon l'une quelconque des revendications précédentes, dans lequel les dimensions de particule de lipoprotéine sont définies sur la base d'expériences de RMN, de CLHP, d'électrophorèse sur gel à gradient ou par microscopie à force atomique.

5. Procédé *in vitro* selon l'une quelconque des revendications précédentes, dans lequel au moins l'un parmi les déplacements chimiques, la largeur, et le coefficient de diffusion est fixe et au moins l'intensité de signal de la lorentzienne centrale ($h_2$) est utilisée comme paramètre libre.

6. Procédé *in vitro* selon l'une quelconque des revendications 1 à 5, dans lequel les fonctions modèles utilisées pour construire les modèles de régression sont des triplets de fonctions lorentziennes de la forme :

$$Triplet_j = Lorentzienne(h_{1j}, f_j - f_{0j}, w_j, D_j) + Lorentzienne(h_{2j}, f_j, w_j, D_j)$$
$$+ Lorentzienne(h_{3j}, f_j + f_{0j}, w_j, D_j).$$

7. Procédé *in vitro* selon l'une quelconque des revendications précédentes, dans lequel la pluralité d'échantillons utilisée pour construire les modèles de régression comprend au moins 100 échantillons et un pourcentage d'au moins 9% des échantillons correspond à des individus ayant un profil de diabète sucré et au moins 25 % de ces patients ayant un profil de dyslipidémie athérogène.

8. Procédé *in vitro* selon l'une quelconque des revendications précédentes, dans lequel le coefficient de diffusion des fonctions modèles est estimé à partir de la dimension de particule de lipoprotéine au moyen de l'équation de Stokes Einstein

$$D = \frac{kT}{6\pi\eta R_H}$$

$k$ (J K$^{-1}$) étant la constante de Boltzmann, $T$ (K) la température, $\eta$ (Pa s) la viscosité et $R_H$ (Å) la dimension de particule de lipoprotéine.

9. Procédé *in vitro* selon l'une quelconque des revendications précédentes, le procédé comprenant en outre la correction des coefficients de diffusion estimés pour prendre en compte des effets de dilution, sur la base d'une relation entre la surface de RMN et le coefficient de diffusion obtenue pour plusieurs dilutions d'un échantillon, la somme de la concentration du cholestérol total et des triglycérides dudit échantillon étant supérieure à 300 mg/dL.

10. Procédé *in vitro* selon l'une quelconque des revendications précédentes, comprenant en outre la détermination d'un ou plusieurs éléments parmi : la dimension moyenne de fractions de particule de lipoprotéine, la dimension moyenne de sous-classes de particule de lipoprotéine, la concentration de particule de lipoprotéine de fraction et/ou sous-classe, la concentration en lipide d'au moins une fraction de particule de lipoprotéine et/ou la concentration en lipide d'au moins une sous-classe de particule de lipoprotéine.

11. Procédé *in vitro* selon la revendication 10, dans lequel la dimension moyenne de particule d'une fraction de particule de lipoprotéine est déterminée comme suit :

$$Dimension\left(\mathring{A}\right) = \frac{\sum_{j=1}^{n} R_j * PN_j}{\sum_{j=1}^{n} PN_j},$$

n étant le nombre de sous-classes de particule de lipoprotéine comprises dans la fraction de particule de lipoprotéine, $R$(Å) étant la dimension de particule de lipoprotéine et $PN_j$ étant le nombre de particule pour ladite dimension de particule de lipoprotéine, le nombre de particule *(PN)* pour une lipoprotéine *j* étant déterminé comme suit :

$$PN_j \propto \frac{A_j}{R_j^3}$$

$A$(ua) étant la surface associée à chaque fonction modèle.

12. Procédé *in vitro* selon l'une quelconque des revendications précédentes, dans lequel la surface associée à une fonction modèle de lipoprotéine est corrigée pour tenir compte uniquement de la contribution des lipides compris dans le noyau de particule de lipoprotéine.

13. Procédé *in vitro* selon la revendication 12, dans lequel la surface corrigée (*A'*) est déterminée à l'aide de l'expression suivante :

$$A' = A \cdot \frac{(9 \cdot (R-s)^3)}{[(9 \cdot (R-s)^3) + 6 \cdot p \cdot (R^3 - (R-s)^3)]}$$

$A$(ua) et $R$(Å) étant respectivement la surface et la dimension de particule de lipoprotéine associées à chaque fonction modèle, $s$(Å) étant l'épaisseur d'enveloppe de particule de lipoprotéine et $p$ étant le rapport de la masse de protéine dans l'enveloppe de la particule par rapport à la masse totale dans l'enveloppe de particule.

**FIG. 1**

**FIG. 2**

**FIG. 3**

**FIG. 4**

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9303450 A1
- WO 2005043171 A1
- WO 2005119285 A1

### Non-patent literature cited in the description

- **K.R. KULKARNI et al.** Quantification of cholesterol in all lipoprotein classes by the VAP-II method. *Journal of Lipid Research,* 1994, vol. 35, 159-168
- **G.R. WARNICK et al.** Polyacrylamide gradient gel electrophoresis of lipoprotein subclasses. *Clinics in Laboratory Medicine,* 2006, vol. 26, 803
- **M. OKAZAKI et al.** Component analysis of HPLC profiles of unique lipoprotein subclass cholesterols for detection of coronary artery disease. *Clinical Chemistry,* 2006, vol. 52, 2049-2053
- **M.P. CAULFIELD et al.** Direct determination of lipoprotein particle sizes and concentrations by ion mobility analysis. *Clinical Chemistry,* 2008, vol. 54, 1307-1316
- **M. ALA-KORPELA et al.** 1H NMR-based absolute quantification of human lipoproteins and their lipid contents directly from plasma. *Journal of Lipid Research,* 1994, 2292-2304
- **R. MALLOL et al.** Particle size measurement of lipoprotein fractions using diffusion ordered NMR spectroscopy. *Analytical and Bioanalytical Chemistry,* 2012, vol. 402, 2407-2415
- **MALLOL R. et al.** *Anal Bioanal Chem,* 2012, vol. 402, 2407-2415
- **MALLOL R. et al.** *Progr. Nucl. Mag. Res. Spectr.,* 2012, vol. 70, 1-24
- **STAHLMAN M. et al.** *BBA - Mol. Cell Biol. Lipids.,* 2013, vol. 1831, 1609-1617
- Quantitation, Composition and Metabolism. **SKIPSKI, V.P.** Blood Lipids and Lipoproteins. Wiley-Interscience, 1972, 471-483
- **SNIDERMAN, A. ; P. O. KWITEROVICH.** Update on the detection and treatment of atherogenic low-density lipoproteins. *Current opinion in endocrinology, diabetes, and obesity,* 2013, vol. 20, 140-147
- **MACKEY, R. H. ; P. GREENLAND ; D. C. GOFF, JR. ; D. LLOYD-JONES ; C. T. SIBLEY ; S. MORA.** High-density lipoprotein cholesterol and particle concentrations, carotid atherosclerosis, and coronary events: MESA (multi-ethnic study of atherosclerosis). *J Am CollCardiol,* 2012, vol. 60, 508-516
- **WURTZ, P. ; J. R. RAIKO ; C. G. MAGNUSSEN ; P. SOININEN ; A. J. KANGAS ; T. TYNKKYNEN ; R. THOMSON ; R. LAATIKAINEN ; M. J. SAVOLAINEN ; J. LAURIKKA.** High-throughput quantification of circulating metabolites improves prediction of subclinical atherosclerosis. *European Heart Journal,* 2012, vol. 33, 2307-2316